(19) Europäisches Patentamt
European Patent Office
Office européen des brevets

(11) Veröffentlichungsnummer: **0 168 737**
**A2**

# (12) EUROPÄISCHE PATENTANMELDUNG

(21) Anmeldenummer: **85108421.0**

(22) Anmeldetag: **08.07.85**

(51) Int. Cl.[4]: **C 07 D 215/56**, C 07 C 57/52, C 07 C 121/76, C 07 C 121/78

(30) Priorität: **18.07.84 DE 3426483**

(43) Veröffentlichungstag der Anmeldung: **22.01.86 Patentblatt 86/4**

(84) Benannte Vertragsstaaten: **AT BE CH DE FR GB IT LI NL SE**

(71) Anmelder: **BAYER AG, Konzernverwaltung RP Patentabteilung, D-5090 Leverkusen 1 Bayerwerk (DE)**

(72) Erfinder: **Grohe, Klaus, Dr., Am Wasserturm 10, D-5068 Odenthal (DE)**

## (54) Verfahren zur Herstellung von halogenierten Chinoloncarbonsäuren.

(57) Verfahren zur Herstellung von halogenierten Chinoloncarbonsäuren der Formel

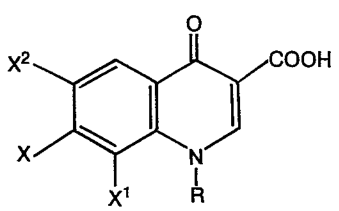

durch Cyclokondensierung von 2-Benzoyl-3-amino-acryl-nitrilen der Formel

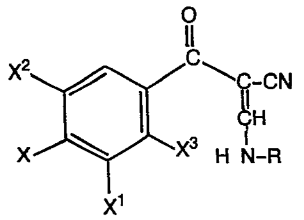

und Verseifung der dabei entstehenden Nitrile.

Die Verbindungen sind wertvolle Zwischenprodukte für die Herstellung antibakterieller Substanzen.

EP 0 168 737 A2

BAYER AKTIENGESELLSCHAFT 5090 Leverkusen, Bayerwerk
Konzernverwaltung RP
Patentabteilung Si/Th-c

# Verfahren zur Herstellung von halogenierten Chinoloncarbonsäuren

Die vorliegende Erfindung betrifft ein Verfahren zur Herstellung von halogenierten Chinoloncarbonsäuren, die wertvolle Zwischenprodukte für die Synthese von hochwirksamen antibakteriellen Arzneimitteln sind.

Es wurde gefunden, daß man die halogenierten Chinoloncarbonsäuren der Formel I

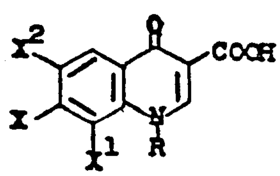

I

in der

R Alkyl mit 1-3 Kohlenstoffatomen, 2-Fluorethyl, Phenyl, Methoxy oder Cyclopropyl,

X Halogen, vorzugsweise Chlor oder Fluor,

$X^1$ Wasserstoff oder Halogen, vorzugsweise Fluor, und

$X^2$ Wasserstoff, Halogen, vorzugsweise Fluor, bedeutet,

erhält, wenn man die entsprechenden 2-Benzoyl-3-amino-acrylnitrile der Formel III

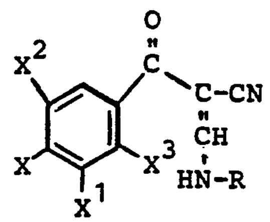

III

in der R, X, $X^1$ und $X^2$ die oben angegebene Bedeutung haben und $X^3$ für Halogen, insbesondere für Chlor oder Fluor steht, cyclokondensiert und die dabei entstehenden Chinoloncarbonsäurenitrile der Formel II

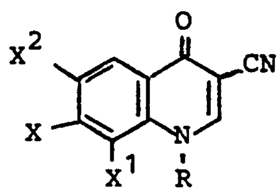

II

verseift.

Die genannte Cyclokondensation wird vorteilhaft unter Verwendung von Natrium- oder Kaliumalkoholaten mit 1-4 C-Atomen im Alkoholteil, z.B. Na-Methylat, sowie Natriumhydrid und besonders bevorzugt von Natrium- oder Kaliumcarbonat durchgeführt.

Die Verseifung kann im alkalischen oder im sauren Medium durchgeführt werden.

Die als Ausgangsprodukte verwendeten Chinoloncarbonsäurenitrile werden wie folgt hergestellt:

Man acyliert mit den halogenierten Benzoylhalogeniden (1) in Gegenwart von wasserfreiem Aluminiumchlorid Acetylen (2) zu den Chlorvinylketonen (3), die mit Hydroxylamin (4) zu den Isoxazolen (5) cyclisiert werden. Mit Alkalialkoholat gehen diese unter Ringöffnung in die gewünschten Benzoylacetonitrile (6) über. Die Umsetzung von (6) mit o-Ameisensäuretrimethylester führt zu den 2-Benzoyl-3-methoxy-acrylnitrilen (7), die mit primären Aminen (8) zu den 2-Benzoyl-3-amino-acrylnitrilen (9) = III reagieren. Die Cyclokondensation von (9) zu den Chinoloncarbonsäurenitrilen II erfolgt z.B. mit Kaliumcarbonat in Dimethylformamid bei 150°C.

Die Verseifung der so erhaltenen Chinoloncarbonsäurenitrilen II führt dann erfindungsgemäß zu den Carbonsäuren I.

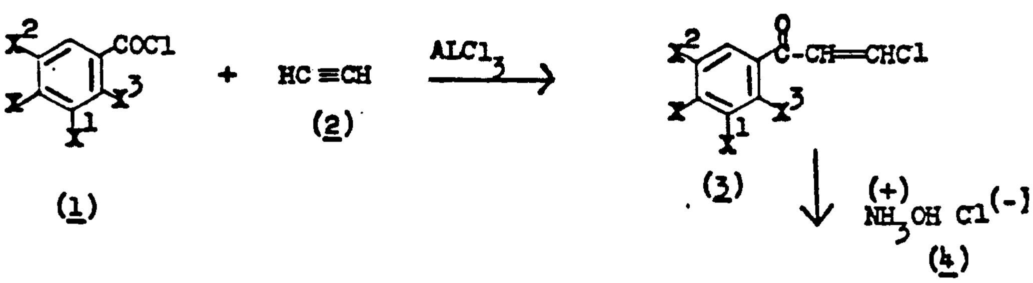


(1) (2) (3) (4)

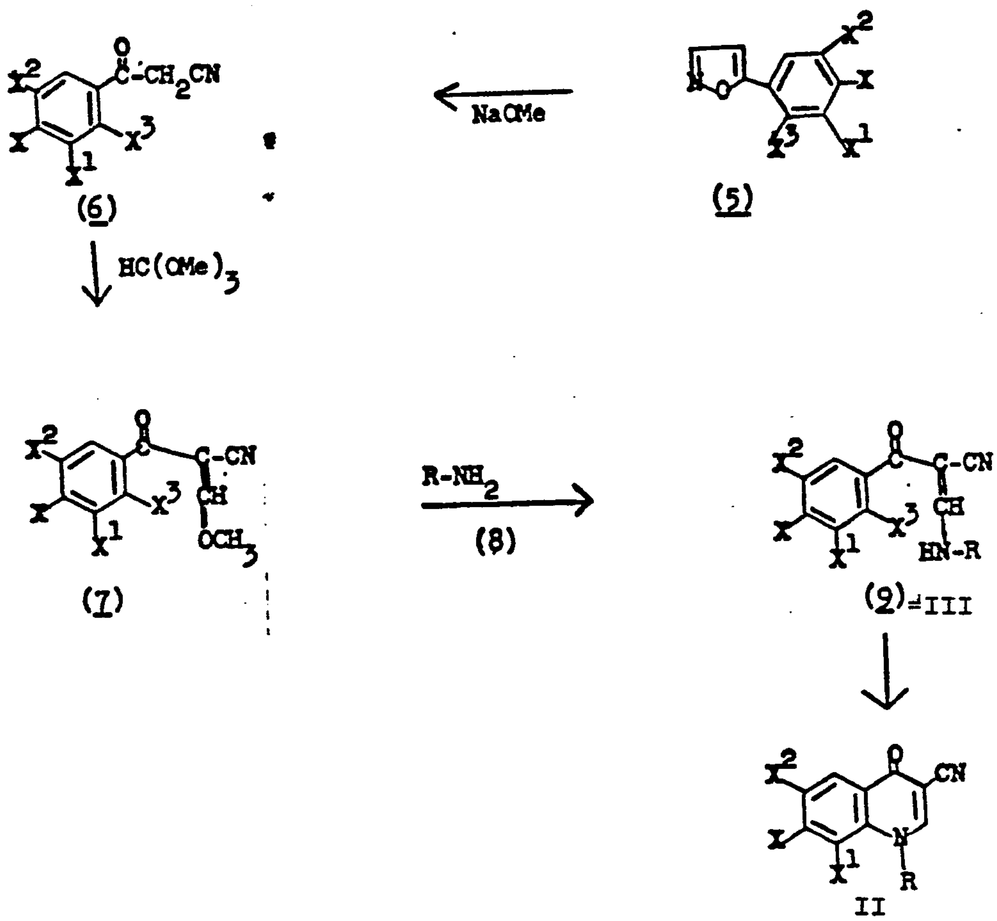


(6) (5) (7) (9)=III II

In diesen Formeln steht $X^3$ für Halogen, vorzugsweise für Chlor oder Fluor.

Die Benzoylacetonitrile (6) können auch auf andere Weise, z. B. durch Umsetzung von Benzoesäureestern (10) mit Acetonitril in Gegenwart einer Base, wie z. B. Natriumhydrid oder K-tert.-butanolat, erhalten werden.

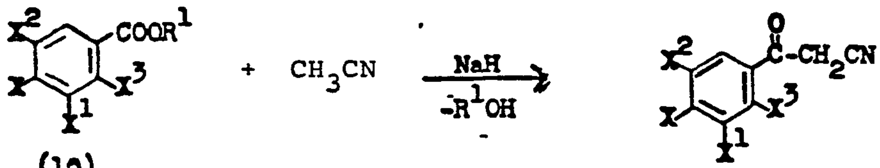


(10) (6)

Dabei bedeutet $R^1$ einen Alkylrest mit 1 bis 4 Kohlenstoffatomen, vorzugsweise Methyl oder Ethyl.

Die Benzoylchloride (1) sind bekannt, z. B. aus DE-OS 3 142 856, oder können aus den bekannten Carbonsäuren mit Thionylchlorid in üblicher Weise hergestellt werden. Die Benzoesäureester (10) werden aus den entsprechenden Carbonsäuren oder Carbonsäurechloriden und den entsprechenden Alkoholen erhalten.

Als Beispiele für die Benzoylchloride (1) seien genannt: 2,4-Dichlor-5-fluor-benzoylchlorid, 2,4,5-Trifluorbenzoylchlorid, 2,3,4,5-Tetrafluorbenzoylchlorid, 2,4,5-Trichlorbenzoylchlorid, 2,3,4,5-Tetrachlorbenzoylchlorid, 2,4,5-Trifluor-3-chlor-benzoylchlorid.

Es ist bereits bekannt geworden, daß man halogenierte Benzoylchloride über die entsprechenden Benzoylmalonester, Benzoylessigester, 2-Benzoyl-3-alkoxyacylsäureester und 2-Benzoyl-3-alkylaminoacrylsäureester in Chinoloncarbonester bzw. Chinoloncarbonsäuren überführen kann (DE-OS 3 142 854).

Dieses Verfahren weist jedoch einige Nachteile auf. So ist die selektive Verseifung der Benzoylmalonester zu den entsprechenden Benzoylessigestern häufig mit Ausbeuteverlusten verbunden. Neben dem gewünschten Benzoylessigester werden wechselnde Mengen Acetophenon erhalten.

Die erfindungsgemäß hergestellten halogenierten Chinoloncarbonsäuren können in hochwirksame antibakterielle Arzneimittel übergeführt werden. So entsteht z. B. bei der Umsetzung von 7-Chlor-1-cyclopropyl-6-fluor-1,4-dihydro-4-oxo-3-chinolincarbonsäure mit Piperazin das bekannte Breitbandchemotherapeutikum Ciprofloxacin (DE-OS 3 142 854):

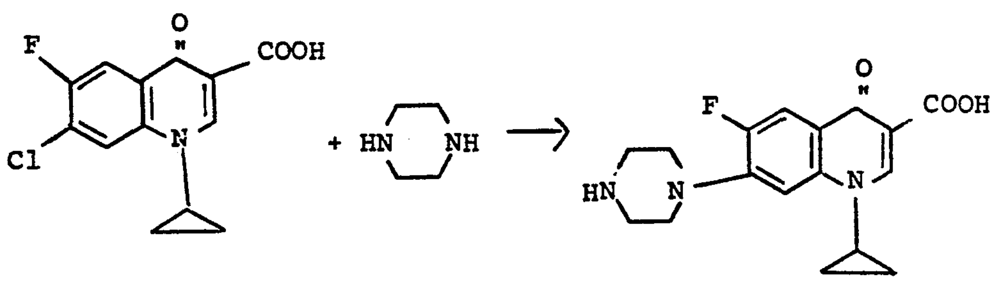

Ciprofloxacin

Beispiel 1:

I 2,4-Dichlor-5-fluor-benzoyl-acetonitril

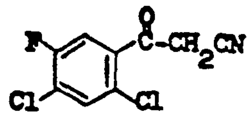

1) 2-Chlorvinyl-(2,4-dichlor-5-fluor-phenyl)-keton

Eine Suspension von 73 g wasserfreiem Aluminiumchlorid und 200 ml trockenem 1,2-Dichlorethan wird unter Eiskühlung und Rühren bei 0 - 10°C tropfenweise mit 113,75 g (0,5 Mol) 2,4-Dichlor-5-fluorbenzoylchlorid versetzt. Dann wird 6,5 Stunden Acetylen bei 40 - 50°C eingeleitet. Das Reaktionsgemisch wird auf Eis gegossen, mit Methylenchlorid die organische Phase aufgenommen und mit Methylenchlorid nachextrahiert. Nach dem Trocknen mit Natriumsulfat wird das Lösungsmittel im Vakuum abdestilliert. Man erhält 121,7 g 2-Chlorvinyl-(2,4-dichlor-5-fluor-phenyl)-keton als Rohprodukt vom Schmelzpunkt 65 - 70°C. Die Destillation im Feinvakuum liefert 99,8 g (78,7 %) reines Produkt vom Siedepunkt: 124 - 128°C/0,1 mbar und Schmelzpunkt 71 - 73°C.

2) 5-(2,4-Dichlor-5-fluor-phenyl)-isoxazol

Ein Gemisch von 57,6 g 2-Chlorvinyl-(2,4-dichlor-5-fluor-phenyl)-keton, 16,4 g Hydroxylamin-hydrochlorid und 150 ml Methanol wird 6 Stunden unter Rückfluß zum Sieden erhitzt, wobei sich nach etwa

einer Stunde ein Niederschlag abscheidet. Dieser wird kalt abgesaugt, mit wenig Methanol gewaschen und im Vakuum bei 50°C getrocknet. Man erhält 33,3 g (63,2 %) 5-(2,4-Dichlor-5-fluor-phenyl)-isoxazol vom Schmelzpunkt 112-113°C, das nach NMR-spektroskopischen Untersuchungen ca. 6 Mol % eines Isomeren enthält.

3) 2,4-Dichlor-5-fluor-benzoylacetonitril

Zu einer Lösung von 2,3 g (0,1 g-Atom) Natrium in 100 ml absolutem Ethanol gibt man unter Eiskühlung und Rühren portionsweise 23,2 g (0,1 Mol) 5-(2,4-Dichlor-5-fluor-phenyl)-isoxazol in ca. 30 Minuten. Dann rührt man 1 Stunde bei Raumtemperatur, destilliert den Alkohol im Vakuum ab, löst den Rückstand in ca. 1 Liter warmen Wasser, extrahiert zweimal mit Methylenchlorid, säuert die wäßrige Phase mit 10-proz. Salzsäure (~38 ml) auf pH=4 an, saugt den Niederschlag kalt ab und trocknet im Vakuum bei 50°C. Es werden 21,7 g (93 %) 2,4-Dichlor-5-fluor-benzoylacetonitril vom Schmelzpunkt 98-99°C erhalten.

Kondensationsverfahren

a) Zu einer Suspension von 6,1 g 80 %-igem Natriumhydrid in 100 ml wasserfreiem Tetrahydrofuran tropft man unter Rückfluß in ca. 1 Stunde ein Gemisch von 47,4 g 2,4-Dichlor-5-fluorbenzoesäureethylester und 8,2 g wasserfreiem Acetonitril. Man erhitzt eine weitere Stunde

unter Rückfluß zum Sieden, kühlt auf Raumtemperatur ab und gibt 150 ml Ether zu. Der Niederschlag wird abgesaugt, in 50 ml Eiswasser gelöst und mit 10 %-iger Salzsäure auf pH=6 angesäuert. Man saugt kalt ab, wäscht mit Eiswasser nach und trocknet im Vakuum bei 40°C. Es werden 16 g 2,4-Dichlor-5-fluor-benzoylacetonitril vom Schmelzpunkt 96-98°C erhalten.

b) Ein Gemisch von 47,4 g (0,2 Mol) 2,4-Dichlor-5-fluor-benzoesäureethylester, 8,5 g wasserfreiem Acetonitril und 100 ml absol. tert.-Butanol wird portionsweise mit 22,4 g (0,2 Mol) Kalium-tert.-butanolat versetzt. Nachdem die leicht exotherme Reaktion abgeklungen ist wird 4 Stunden refluxiert, das Lösungsmittel im Vakuum abgezogen und der Rückstand in $H_2O/CH_2Cl_2$ aufgenommen. Die wäßrige Phase wird unter Eiskühlung mit 20 ml Eisessig versetzt, der Niederschlag abgesaugt, mit Wasser gewaschen und im Vakuum bei 50°C getrocknet. Es werden 25,7 g (55,4 %) 2,4-Dichlor-5-fluoracetonitril vom Schmelzpunkt 96-99°C erhalten.

Der als Ausgangsmaterial verwendete 2,4-Dichlor-5-fluor-benzoesäureethylester wird wie folgt erhalten:

84 g 2,4-Dichlor-5-fluor-benzoylchlorid werden bei 50 bis 60°C zu 250 ml wasserfreiem Ethanol getropft, wobei die Temperatur durch gelegentliches Kühlen

mit Eiswasser gehalten wird. Dann wird noch ca. 1 Stunde refluxiert, der Überschüssige Alkohol abdestilliert und der Rückstand im Vakuum fraktioniert. Bei 132-135°C / 8 mbar werden 84,9 g (~97 %) 2,4-Dichlor-5-fluor-benzoesäureethylester erhalten.

II <u>2-(2,4-Dichlor-5-fluor-benzoyl)-3-methoxyacrylnitril</u>

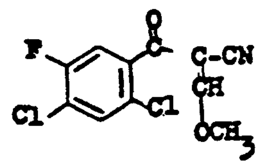

Ein Gemisch von 21 g 2,4-Dichlor-5-fluor-benzoyl-acetonitril, 19,2 g o-Ameisensäuremethylester und 23,1 g Acetanhydrid wird 3 Stunden bei einer Badtemperatur von 150 °C refluxiert. Dann werden die flüchtigen Bestandteile im Wasserstrahlvakuum und zuletzt im Feinvakuum bei 120 °C Badtemperatur abdestilliert. Es werden 24,5 g (99 %) 2-(2,4-Dichlor-5-fluor-benzoyl)-3-methoxy-acrylnitril erhalten. Eine Probe aus Toluol umkristallisiert liefert farblose Kristalle vom Schmelzpunkt 159 °C.

III <u>2-(2,4-Dichlor-5-fluor-benzoyl)-3-cyclopropyl-amino-acrylnitril</u>

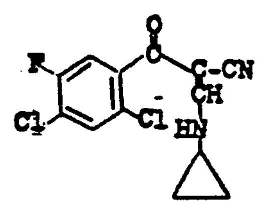

Eine Lösung von 22,5 g 2-(2,4-Dichlor-5-fluor-benzoyl)-3-methoxy-acrylnitril in 80 ml Ethanol wird unter Eiskühlung und Rühren, tropfenweise mit 5,6 g Cyclopropylamin versetzt. Man rührt 25 Minuten bei Raumtemperatur und erhitzt dann 15 Minuten zum Sieden. Das Lösungsmittel wird im Vakuum entfernt und der Rückstand aus Ethanol / Leichtbenzin umkristallisiert. Es werden 17,7 g 2-(2,4-Dichlor-5-fluor-benzoyl)-3-cyclopropyl-amino-acylnitril vom Schmelzpunkt 95°C erhalten.

IV 7-Chlor-1-cyclopropyl-6-fluor-1,4-dihydro-4-oxo-3-chinolincarbonsäurenitril

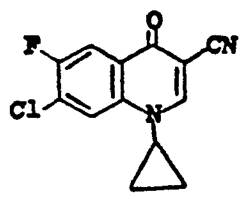

17,7 g 2-(2,4-Dichlor-5-fluor-benzoyl)-3-cyclopropylamino-acrylnitril, 9 g wasserfreies Kaliumcarbonat und 60 ml wasserfreies Dimethylformamid werden 2 Stunden auf 150-160°C erhitzt. Man gießt das heiße Gemisch auf Eis, saugt den Niederschlag ab und wäscht ihn gut mit Wasser. Nach dem Trocknen

im Vakuum bei 100°C werden 14,9 g reines 7-Chlor-1-cyclopropyl-6-fluor-1,4-dihydro-4-oxo-3-chinolin-carbonsäurenitril vom Schmelzpunkt 239 - 241°C erhalten.

V **7-Chlor-1-cyclopropyl-6-fluor-1,4-dihydro-4-oxo-3-chinolincarbonsäure.**

2,6 g (0,01 Mol) 7-Chlor-1-cyclopropyl-6-fluor-1,4-dihydro-4-oxo-3-chinolincarbonsäurenitril werden mit 12 ml Wasser und 10 ml konz. Schwefelsäure 1,5 Stunden unter Rückfluß zum Sieden erhitzt. Dabei beträgt die Badtemperatur 170 - 180°C, die Innentemperatur 132 - 134°C. Man gießt in 30 ml Eiswasser, saugt den Niederschlag ab, wäscht gut mit Wasser und löst heiß in 30 ml 10-proz. Natronlauge. Die Lösung wird heiß filtriert, mit 100 ml heißem Wasser nachgespült, mit Eis gekühlt und mit halbkonz. Salzsäure auf pH= 1 angesäuert. Der hellgelbe Niederschlag wird abgesaugt, mit Wasser gewaschen und im Vakuum bei 100°C getrocknet. Ausbeute: 2,2 g 7-Chlor-1-cyclopropyl-6-fluor-1,4-dihydro-4-oxo-3-chinolincarbonsäure vom Schmelzpunkt 234-236°C. Nach Umkristallisation aus Glykolmonomethylether werden 1,8 g (64,6 %) hellgelbe Kristalle vom Schmelzpunkt 240-241°C erhalten.

Beispiel 2:

I 2,4-Dichlor-benzoylacetonitril

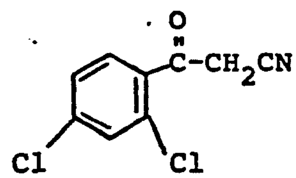

Zu einer Suspension von 6,1 g 80 %-igem Natriumhydrid in 100 ml wasserfreiem Tetrahydrofuran tropft man unter Rückfluß in ca. 1 Stunde ein Gemisch von 43,8 g 2,4-Dichlor-benzoesäureethylester und 8,2 g wasserfreiem Acetonitril. Man erhitzt eine weitere Stunde unter Rückfluß zum Sieden, kühlt auf Raumtemperatur ab und gibt 150 ml Ether zu. Der Niederschlag wird abgesaugt, in 50 ml Eiswasser gelöst und mit 10 %-iger Salzsäure auf pH= 6 angesäuert. Man saugt kalt ab, wäscht mit Eiswasser nach und trocknet im Vakuum bei 40°C. Es werden 12 g 2,4-Dichlor-benzoylacetonitril vom Schmelzpunkt 96-98°C erhalten.

II 2-(2,4-Dichlor-benzoyl)-3-methoxyacrylnitril

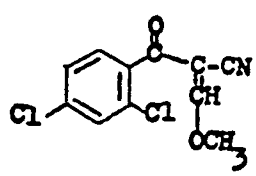

Ein Gemisch von 8,8 g 2,4-Dichlor-benzoylacetonitril, 69,2 g o-Ameisensäuremethylester und 10,5 g

Acetanhydrid wird 3 Stunden bei einer Badtemperatur von 150°C refluxiert. Dann werden die flüchtigen Bestandteile im Vakuum und zuletzt im Feinvakuum bei 120°C Badtemperatur abdestilliert. Es werden 11 g 2-(2,4-Dichlor-benzoyl)-3-methoxy-acrylnitril als zähes Öl erhalten.

III 2-(2,4-Dichlor-benzoyl)-3-cyclopropyl-amino-acrylnitril

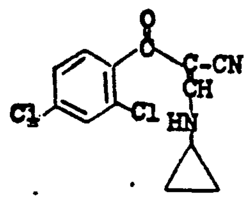

Eine Lösung von 11 g 2-(2,4-Dichlor-benzoyl)-3-methoxy-acrylnitril in 40 ml Ethanol wird unter Eiskühlung und Rühren, tropfenweise mit 2,5 g Cyclopropylamin versetzt. Man rührt 25 Minuten bei Raumtemperatur und erhitzt dann 15 Minuten zum Sieden. Das Lösungsmittel wird im Vakuum entfernt und der Rückstand aus Ethanol / Leichtbenzin umkristallisiert. Es werden 10,5 g 2-(2,4-Dichlorbenzoyl)-3-cyclopropylamino-acrylnitril vom Schmelzpunkt 93 - 94°C erhalten.

IV 7-Chlor-1-cyclopropyl-1,4-dihydro-4-oxo-3-chinolincarbonsäurenitril

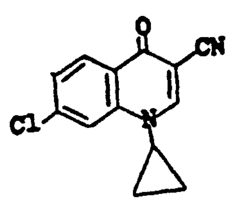

10,2 g 2-(2,4-Dichlor-benzoyl)-3-cyclopropylamino-acrylnitril, 6,1 g wasserfreies Kaliumcarbonat und 40 ml wasserfreies Dimethylformamid werden 2 Stunden auf 150 - 160°C erhitzt. Man gießt das heiße Gemisch auf Eis, saugt den Niederschlag ab und wäscht ihn gut mit Wasser. Nach dem Trocknen im Vakuum bei 100°C werden 8,3 g rohes 7-Chlor-1-cyclopropyl-1,4-dihydro-4-oxo-3-chinolincarbonsäurenitril erhalten. Nach Umkristallisation aus Acetonitril schmelzen die hellgelben Kristalle bei 270 - 272°C. Ausbeute: 7 g

V 7-Chlor-1-cyclopropyl-1,4-dihydro-4-oxo-3-chinolincarbonsäure.

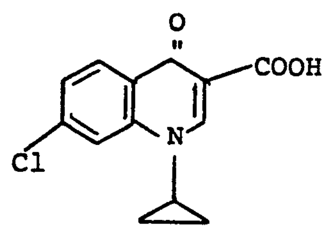

2,5 g (0,01 Mol) 7-Chlor-1-cyclopropyl-1,4-dihydro-4-oxo-3-chinolincarbonsäurenitril werden mit 12 ml Wasser und 10 ml konz. Schwefelsäure 1,5 Stunden unter Rückfluß zum Sieden erhitzt. Dabei beträgt die Badtemperatur 170 - 180°C, die Innentemperatur 132 - 134°C. Man gießt in 30 ml Eiswasser, saugt den Niederschlag ab, wäscht gut mit Wasser und löst heiß in 30 ml 10 Prozent Natronlauge. Die Lösung wird heiß filtriert, mit 100 ml heißem

Wasser nachgespült, mit Eis gekühlt und mit halbkonz. Salzsäure auf pH= 1 angesäuert. Der hellgelbe Niederschlag wird abgesaugt, mit Wasser gewaschen und im Vakuum bei 100°C getrocknet. Ausbeute: 2,4 g 7-Chlor-1-cyclopropyl-1,4-dihydro-4-oxo-3-chinolincarbonsäure vom Schmelzpunkt 307 - 308°C.

Patentansprüche

1. Verfahren zur Herstellung von halogenierten Chinoloncarbonsäuren der Formel I

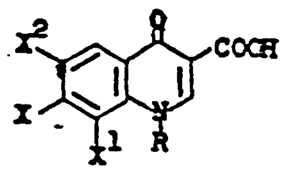

I

in der

R Alkyl mit 1-3 Kohlenstoffatomen, 2-Fluorethyl, Phenyl, Methoxy oder Cyclopropyl,

X Halogen, vorzugsweise Chlor oder Fluor,

$X^1$ Wasserstoff oder Halogen, vorzugsweise Fluor, und

$X^2$ Wasserstoff, Halogen, vorzugsweise Fluor, bedeutet,

dadurch gekennzeichnet, daß man 2-Benzoyl-3-aminoacrylnitrile der Formel III

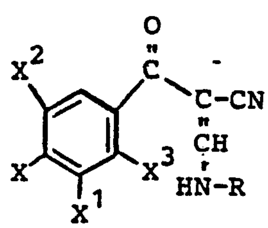

III

in der R, X, $X^1$ und $X^2$ die oben angegebene Bedeutung haben und $X^3$ für Halogen, insbesondere für Chlor oder Fluor steht, cyclokondensiert und die dabei entstehenden Chinoloncarbonsäurenitrile der Formel II

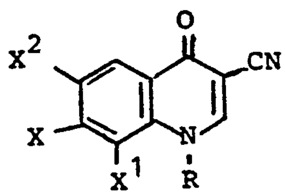

II

verseift.

2. Verfahren nach Anspruch 1, dadurch gekennzeichnet, daß man die Cyclokondensation in Gegenwart von Natrium- oder Kaliumcarbonat durchführt.

3. 2,4-Dichlor-5-fluorbenzoesäureethylester.

4. 2,4-Dichlor-5-fluorbenzoylacetonitril.

5. 2-(2,4-Dichlor-5-fluorbenzoyl)-3-methoxyacrylnitril.

6. 2-(2,4-Dichlor-5-fluorbenzoyl)-3-cyclopropyl-amino-acrylnitril.

7. 7-Chlor-1-cyclopropyl-6-fluor-1,4-dihydro-4-oxo-3-chinolincarbonsäurenitril.

8. 2-(2,4-Dichlor-benzoyl)-3-methoxyacrylnitril.

9. 2-(2,4-Dichlor-benzoyl)-3-cyclopropyl-amino-acrylnitril.

10. 7-Chlor-1-cyclopropyl-1,4-dihydro-4-oxo-3-chinolin-carbonsäurenitril.